# EUROPEAN PATENT SPECIFICATION

(11) **EP 1 043 393 B1**
(45) Date of publication and mention of the grant of the patent: **17.11.2004**
(21) Application number: 00107610.8
(22) Date of filing: 07.04.2000
(51) Int. Cl.: C12N 9/08, C07K 1/14

(54) **Method for the purification of lactoperoxidase**
Methode für Lactoperoxidase reinigung
Méthode de purification de lactoperoxidase

(30) Priority: 09.04.1999 IT BO990165
(43) Date of publication of application: 11.10.2000
(73) Proprietor: Universita' degli studi di Bologna, 40121 Bologna (IT)
(72) Inventor: Pifferi, Piergiorgio, Bologna (IT); Villalonga, Aldemaro, Venezuela (VE)
(74) Representative: Negrini, Elena

(56) References cited:
- EP-A- 0 418 704
- EP-A- 0 556 083
- DATABASE WPI Section Ch, Week 198913 Derwent Publications Ltd., London, GB; Class A11, AN 1989-096189 XP002190339 & JP 01 043501 A (NAKAHARA SHOKAI KK), 15 February 1989 (1989-02-15)
- DATABASE WPI Section Ch, Week 199124 Derwent Publications Ltd., London, GB; Class A97, AN 1991-178052 XP002191251 & WO 91 07427 A (NISSEI CHEM IND CO), 30 May 1991 (1991-05-30)

## Description

The present invention relates to the agroindustrial refluent treatment using natural macromolecules in order to extract effectively some useful substances for the food industry.

Particularly the invention refers to a method of liquid treatment deriving from the milk technology for getting cheese and/or ricotta, such as whey, in order to recover and to purify an oxidase enzyme - the lactoperoxidase (LPO).

This enzyme is useful because it provides catalytic and bactericide action on numerous micro-organisms, pathogen and not pathogen, and is important to prolong the storage shelf-life (for instance on the shelves of the shops) of different foods, if it is added as additive or adjuvant in the process, in presence of its substrate.

Generally in the milk of bovine, sheep, goat, and camel the LPO ranges from 1500 to 16.000 UE/Litre. Said enzyme almost completely remains in the whey after the milk coagulation to get cheeses or in the liquid to get the ricotta by means of acidification and heating the rennet whey at the temperature not higher than 60°C.

Recovering said enzyme has been effected by expensive matrix, such as the insoluble carboxymethylcellulose, and by using affinity chromatography with dextran derivative, obtained by reaction with iminodiacetic acid and by saturation with metals, such as copper.

Said polymers are notably expensive and not always, such as in the case of gel-carboxymethylcellulose, allow to get high purification ratio defined as the ratio of catalytic activity /protein in the volume unit.

Document EP 0 556 083 discloses a purification method of lactoperoxidase from milk or whey, where cellulose, sulfonated Cellulofine or sulfonated Chitopearl is mixed with cheese Whey.

The main object of the present invention is to propose a method for treating whey of acid or rennet milk coagulation or the residual liquids of the ricotta products, with macromolecule of natural origin such as the pectin or macromolecules from agroindustrial discards containing pectin, to recover totally the lactoperoxidase enzyme.

Another object of the invention is to propose an adsorbent, being washed sequentially with buffer at definite pH value, which allows to recover the lactoperoxidase by notable removing the extraneous protein, and consequently recovery of the purified LPO and of the adsorbent, which can be used again in subsequent cycles.

Further object is to propose a method which can be carried out easily and economically.

The above mentioned purposes are obtained in conformity with the contents of the claims.

The method according to the invention is fit to remove totally or at least partially the lactoperoxidase from the whey of milk for cheese-making or from the ricotta production, to which is mixed an adsorbent, powder or granule matter, consisting of pectin at different esterification degrees or matrixes pectin-lignin-cellulose containing pectin besides cellulose, hemicellulose, lignin, after saponification to reduce the pectin esterification degree.

The liquids, which are going to be treated in the pH range 4,0-8,0, are mixed with pectin, pure or bonded with macromolecule, dissolved in phosphate buffer in the range of pH 4,0 to 8,0 at a concentration of 1-20 gram/litre, at the temperature between 2-30°C for up to 4 hours, and then after centrifugation at 4°C for 30 minutes, the precipitate containing the lactoperoxidase will be suspended in 0,02 mol·l⁻¹ phosphate buffer in pH range between 6,0 to 8,5, centrifuged for 30 minutes and re-suspended at least 4 times, while at the fifth time the suspension has a solution of the same pH value, but in 0,25 mol·l⁻¹ chloride sodium. The suspension volume is from 1/5 to 1/10 of the treated solution.

The alimentary liquids consist essentially of cheese making whey obtained by acids or by rennet or of liquid for obtaining ricotta using acids or salts, centrifuged for 30 minutes at 4°C to remove the suspended material, and carried at the pH ranging from pH 4,0 to 8,0 using 1 mol·l⁻¹ citric acid.

The adsorbent includes pectin (for instance of apples, of citrus fruit, of beet) or macromolecule complexes - containing not only pectin, but also cellulose, hemicellulose, lignin, as well as citrus peels, apple marc, beet strips.

The esterification ratio of the pectin or the pectin part is from 95% and 0,05%.

The adsorbent, with granulometry not more than 3 mm, can be added dry into the treating liquids, or be dissolved in aqueous buffer solution at pH 4,0-8,0.

The adsorbent concentration in the food liquid ranges from 100g to 2000g in one hectolitre of liquid mixture.

After 4 hours at a preferred temperature of 20°C the suspension is centrifuged for 60 minutes at 4°C to 10°C at 10.000 rpm.

The sediment obtained from above process, will be re-suspended in 0,02 mol·l⁻¹ phosphate buffer in pH ranging from 6,0 to 8,0 containing a concentration of chloride sodium from 0,05 to 0,40 M, centrifuged, and re-suspended several times.

After centrifugation, the last surnatant contains the LPO, while the adsorbent, ready for a new treatment, is contained in the sediment.

Advantageously the alimentary liquids essentially consist of milk acid whey or using rennet, or mixed, or of the ricotta products directly obtained from milk by acidification, or of the salted or acidified whey and heated for separating the protein from the whey.

Advantageously the whey will be at first centrifuged for eliminating the material in suspension, getting in this way a relative limpid liquid and stratified on the surface with the fat, which is removable.

The adsorbent principally comprises the pectin of apple, citrus, beet, sunflower or the by-product of the agroindustrial productions that contain pectin in addition to other polyoses - cellulose, hemicellulose, lignin - such as apple marc, citrus peels, beet strips.

The esterification degree of the pure pectin or the macromolecule complex, can be varied from 95 to 0,05% of carboxyl groups of the pectin component, preferably around 20-30% esterification degree

The pectin concentration of the final solution is from 0,05 to 1,5%, and the preferred suspension of pectin concentration is 1% w/v in pH ranging from 4,0 to 8,0, preferable at pH4,5, is added in relationship of 1:1 w/v to the milk serum in pH ranging from 4,0 to 7,0, preferably carried at pH 5,5 with 1 mol·l⁻¹ citric acid.

Further particularities are described mostly in the following preferred practice examples carrying out the method for treating the agroindustrial refluent according to the present invention, these following examples are only indicative and not limited.

### Example 1

0,5 litre of milk whey for the Grana -Parmigiano Reggiano cheese production is centrifuged at 4°C for 45 minutes at 10.000 rpm, and acidified in pH 5,0 with 1 mol·l⁻¹ citric acid.

A suspension of apple pectin (6.E.8c) is prepared at 2,0 % w/v in 0,12 mol·l⁻¹ phosphate buffer at pH 6,0. Said 0,50 litre solution is mixed with 0,5 litre whey, stirred for 5 minutes and centrifuged for 45 minutes at 10.000 rpm at 4°C. The surnatant does not contain LPO according to the analysis. The obtained precipitate is suspended in 0,1 litre of phosphate buffer 0,02 mol·l⁻¹ at pH 7,0 and 0,10 mol·l⁻¹ in chloride sodium, centrifuged and the centrifuged is re-suspended in the preceding solution. The cycle is repeated 3 times. At the fifth time the centrifuged washing is effected by suspension in 0,030 mol·l⁻¹ buffer pH 7,0 and 0,30 mol·l⁻¹ NaCl; after centrifugation in the same way, the surnatant contains the LPO with a specific activity of 110 compared with the specific activity 10,9 of the whey, and that is 11 times purified.

### Example 2

1 litre of whey as in the example 1, is acidified at pH 5,50 with 1 mol·l⁻¹ citric acid, is mixed at 20°C with 1 litre solution of apple pectin with 25% esterification degree, at 1% w/v in buffer solution of pH 4,5. After stirring for 1 minute, is centrifuged at 4°C for 45 minutes. The sediment is suspended in 0,2 litres of 0,02 mol·l⁻¹ phosphate buffer at pH 7,7 and 0,15 mol·l⁻¹ in NaCl, re-centrifuged for at least 4 times. The sediment of the fourth centrifugation is suspended in 0,2 litres of 0,02 mol·l⁻¹ phosphate buffer at pH 7,70 and 0,30 mol·l⁻¹ in NaCl. After the fifth centrifugation in the usual way the surnatant is analysed. The specific activity is 138,0 with respect to the initial whey specific activity of 6,50, so showing a purification of 22 times.

### Example 3

A litre of whey deriving from the ricotta production from full-cream milk by acidification with 1 mol·l⁻¹ citric acid up to pH 5,00 after separation on double layer of cotton, is centrifuged as usual, and is mixed with 1 litre of 0,12 mol·l⁻¹ buffer at pH 5,00 of apple pectin at 5 % esterification degree and at 2,0 % w/v. After the re-mixture and the centrifugation according to the previous operation, the precipitate is suspended four times in 0,02 mol·l⁻¹ phosphate buffer at pH 8,00 and in 0,15 mol·l⁻¹ the NaCl, and centrifuged as before. The sediment is re-suspended finally in 0,03 mol·l⁻¹ buffer at pH 8,0 and 0,25 mol·l⁻¹ in NaCl. The surnatant analysis shows a specific activity of 16.

## Claims

1. Method for removing totally or partially the lactoperoxidase from an alimentary dairy liquid comprising:
- to acidify said liquid at a pH ranging from 4,0 to 8,0 with 1 mol·l⁻¹ citric acid;
- at the room temperature, to add the alimentary liquid With an adsorbent, in form of powder or of a buffered suspension having a pH ranging from 4,0 to 8,0;
said method being **characterized in**:
- to use an adsorbent containing pectin;
- to carry out by stirring briefly a liquid mixture with a concentration of 1 00g to 2 000g of adsorbent in one hectolitre of said liquid mixture;
- to centrifuge at 4°C to 10°C for maximum 60 minutes at 10000 rpm the liquid mixture obtaining a surnatant without lactoperoxidase and a complex adsorbent-adsorbed;
- to wash repeatedly said complex adsorbent-adsorbed in phosphate buffer at pH ranging from 6,0 to 8,5 and containing sodium chloride with a concentration ranging from 0,05 M to 0,40 mol·l⁻¹ to sequentially remove extraneous substances and finally
to desorb the lactoperoxidase, with simultaneous recovery of the adsorbent as residue by means of the centrifugation and the lactoperoxidase in the ceritrifuged liquid.

2. Method according to claim 1 **characterized in that** said alimentary dairy liquids essentially consist of whey for cheese-making with acids or rennet, or of liquid for the ricotta production by treating milk with acids, or of the whey, deprived of proteins by means of salting, or by acidification and heating.

3. Method according to claim 1 **characterized in that** said powder or added as suspension, has granules with a diameter non more than 3 mm.

4. Method according to claim 1 **characterized in that** said adsorbent substance comprises the pectin or structures of pectin-lignin-cellulose; in which pectin esterification degree ranges from 95% to 0,05 % of the carboxyl groups of the pectin component.

5. Method according to claim 1 **characterized in that** said adsorbent is obtained from the apple marc.

6. Method according to claim 1 **characterized in that** said powder or granule matter is obtained from the citrus peels.

7. Method according to claim 1 **characterized in that** said powder or granule matter is obtained from exhausted beet strips.

8. Method according to claim 1 **characterized in that** said powder or granule matter consists of homopolymer or heteropolymer pectin.

9. Method according to claim 1 **characterized in that** said powder or granule is consisted of pectin-lignin-cellulose structures.

10. Method according to claim 1 **characterized in that** the whey, before adding the powder substance, centrifuged at 4°C for 45 minutes at 10.000 rpm, is acidified with citric acid up to pH 5,50.

11. Method according to claim 1 **characterized in that** the granular adsorbent is added to the whey, in phosphate buffer 0,12 mol·l⁻¹ at pH 4,50.

12. Method according to claim 1 **characterized in that** the precipitate constituted of adsorbent and adsorbed materials, is treated sequentially, with phosphate buffer 0,02 mol·l⁻¹, at pH ranging from 6,0 to 8,0 and 0,25 mol·l⁻¹ in chloride sodium, to remove the extraneous substances, contaminating the enzyme lactoperoxidase.

13. Method according to claim 1 **characterized in that** after removing the extraneous substances according to the claim 12, the lactoperoxidase is desorbed from the granular adsorbent, by means of the suspension in 0,03 mol·l⁻¹ phosphate buffer at pH 7,7 and with a molarity in NaCl equal to or higher than 0,25.

## Patentansprüche

1. Verfahren zum gänzlichen oder teilweisen Beseitigen der Lactoperoxidase aus einer milchwirtschaftlichen Speiseflüssigkeit, mit
- dem Säuern der Flüssigkeit mit 1 Mol·l⁻¹ Zitronensäure auf einen pH-Wert im Bereich von 4,0 bis 8,0;
- dem Versetzen der Speiseflüssigkeit mit einem Adsorptionsmittel in Form eines Pulvers oder einer gepufferten Suspension mit einem pH-Wert im Bereich von 4,0 bis 8,0 bei Zimmertemperatur;
wobei das Verfahren **dadurch gekennzeichnet ist, dass**:
- ein pektinhaltiges Adsorptionsmittel verwendet wird;
- durch kurzes Umrühren eine flüssige Mischung mit einer Konzentration von 100g bis 2000g des Adsorptionsmittels in einem Hektoliter der flüssigen Mischung hergestellt wird;
- die flüssige Mischung maximal 60 Minuten lang bei 4°C bis 10°C bei 10000 U/min zentrifugiert wird, um eine Schwimmdecke ohne Lactoperoxidase und einen Komplex von Adsorptionsmittel und adsorbiertem Stoff zu erhalten;
- der Komplex von Adsorptionsmittel und adsorbiertem Stoff mehrmals in Phosphatpufferlösung bei einem pH-Wert im Bereich von 6,0 bis 8,5 und mit einem Natriumchloridgehalt mit einer Konzentration im Bereich von 0,05 Mol·l⁻¹ bis 0,40 Mol·l⁻¹ gewaschen wird, um sequentiell Fremdstoffe zu beseitigen, und schließlich
- die Lactoperoxidase unter gleichzeitiger Rückgewinnung des Adsorptionsmittels als Rückstand mit Hilfe der Zentrifugierung und der Lactoperoxidase in der zentrifugierten Flüssigkeit desorbiert wird.

2. Verfahren nach Anspruch 1, **dadurch gekennzeichnet, dass** die milchwirtschaftlichen Speiseflüssigkeiten im wesentlichen aus Molke zur Herstellung von Käse mit Säuren oder Labferment oder aus Flüssigkeit zur Herstellung von Ricotta durch Behandlung von Milch mit Säuren oder aus der Molke nach Befreiung von Proteinen mit Hilfe des Salzens oder durch Säuern und Erhitzen bestehen.

3. Verfahren nach Anspruch 1, **dadurch gekennzeichnet, dass** das Pulver bei Zusetzung als Suspension Körnchen mit einem Durchmesser von höchstens 3 mm aufweist.

4. Verfahren nach Anspruch 1, **dadurch gekennzeichnet, dass** das Adsorptionsmittel das Pektin oder Strukturen von Pektin-Lignin-Cellulose umfasst; in denen der Grad der Pektinveresterung im Bereich von 95 % bis 0,05 % der Carboxylgruppen des Bestandteils Pektin liegt.

5. Verfahren nach Anspruch 1, **dadurch gekennzeichnet, dass** das Adsorptionsmittel aus dem Apfeltreber erhalten wird.

6. Verfahren nach Anspruch 1, **dadurch gekennzeichnet, dass** das Pulver oder das Granulat aus den Zitrusschalen erhalten wird.

7. Verfahren nach Anspruch 1, **dadurch gekennzeichnet, dass** das Pulver oder Granulat aus ausgezogenen Rübenstreifen erhalten wird.

8. Verfahren nach Anspruch 1, **dadurch gekennzeichnet, dass** das Pulver oder Granulat aus homopolymerem oder heteropolymerem Pektin besteht.

9. Verfahren nach Anspruch 1, **dadurch gekennzeichnet, dass** das Pulver oder Granulat aus Pektin-Lignin-Cellulosestrukturen besteht.

10. Verfahren nach Anspruch 1, **dadurch gekennzeichnet, dass** die Molke vor dem Zusetzen des 45 Minuten lang mit 10000 U/min bei 4°C zentrifugierten pulverförmigen Stoffs mit Zitronensäure auf einen pH-Wert von 5,50 gesäuert wird.

11. Verfahren nach Anspruch 1, **dadurch gekennzeichnet, dass** das granulare Absorptionsmittel der Molke in Phosphatpufferlösung von 0,12 Mol/l⁻¹ mit einem pH-Wert von 4,50 zugesetzt wird.

12. Verfahren nach Anspruch 1, **dadurch gekennzeichnet, dass** das aus Adsorptionsmittel und adsorbierten Stoffen bestehende abgeschiedene Produkt sequentiell mit Phosphatpufferlösung von 0,02 Mol/l⁻¹ auf einen pH-Wert von 6,0 bis 8,0 und von 0,25 Mol/l⁻¹ Natriumchlorid behandelt wird, um die das Enzym Lactoperoxidase kontaminierenden Fremdstoffe zu beseitigen.

13. Verfahren nach Anspruch 1, **dadurch gekennzeichnet, dass** nach Beseitigung der Fremdstoffe gemäß Anspruch 12 die Lactoperoxidase mit Hilfe der Suspension in 0,03 Mol/l⁻¹ Phosphatpufferlösung mit einem pH-Wert von 7,7 und mit einer Molarität in NaCl, die gleich oder höher als 0,25 ist, aus dem granularen Absorptionsmittel desorbiert wird.

## Revendications

1. Procédé d'élimination totale ou partielle de la lactoperoxydase à partir d'un liquide laitier alimentaire, comprenant les étapes consistant :
- à acidifier ledit liquide à un pH variant de 4,0 à 8,0 avec de l'acide citrique à 1 mole.l⁻¹ ;
- à ajouter, à la température ambiante, le liquide alimentaire dans un adsorbant sous forme d'une poudre ou d'une suspension tamponnée ayant un pH variant de 4,0 à 8,0 ;
ledit procédé étant **caractérisé en ce que** :
- on emploie un adsorbant contenant de la pectine ;
- on forme par agitation brève, un mélange liquide ayant une concentration de 100 g à 2 000 g d'adsorbant dans un hectolitre dudit mélange liquide ;
- on centrifuge à 4 °C jusqu'à 10 °C pendant au maximum 60 minutes à 10 000 tr/min, le mélange liquide pour obtenir un surnageant sans lactoperoxydase et un adsorbant-adsorbat complexe ;
- on lave de manière répétée ledit adsorbant-adsorbat complexe dans un tampon de type phosphate ayant un pH variant de 6,0 à 8,5 et contenant du chlorure de sodium selon une concentration variant de 0,05 à 0,40 mole.l⁻¹ afin d'éliminer successivement les substances étrangères et désorber finalement la lactoperoxydase en récupérant simultanément l'adsorbant sous forme d'un résidu par l'intermédiaire de la centrifugation, et la lactoperoxydase dans le liquide centrifugé.

2. Procédé selon la revendication 1, **caractérisé en ce que** lesdits liquides alimentaires laitiers consistent essentiellement en petit-lait pour la production de fromage avec des acides ou de la présure, ou en un liquide pour la production de recuite par traitement de lait avec des acides, ou du petit-lait débarrassé des protéines par salage, ou par acidification et chauffage.

3. Procédé selon la revendication 1, **caractérisé en ce que** ladite poudre ou l'additif sous forme de suspension, contient des granulés ayant un diamètre non supérieur à 3 mm.

4. Procédé selon la revendication 1, **caractérisé en ce que** ladite substance adsorbante comprend de la pectine ou des complexes de pectine lignine cellulose ; le degré d'estérification de la pectine variant de 95 % à 0,05 % des groupes carboxy du composant de type pectine.

5. Procédé selon la revendication 1, **caractérisé en ce que** ledit adsorbant est obtenu à partir du marc de pomme.

6. Procédé selon la revendication 1, **caractérisé en ce que** ladite poudre ou matière granulaire est obtenue à partir des zestes de citron.

7. Procédé selon la revendication 1, **caractérisé en ce que** ladite poudre ou ladite matière granulaire est obtenue à partir de pelures de betterave épuisées.

8. Procédé selon la revendication 1, **caractérisé en ce que** ladite poudre ou ladite matière granulaire consiste en pectine homopolymère ou hétéropolymère.

9. Procédé selon la revendication 1, **caractérisé en ce que** ladite poudre ou ladite matière granulaire consiste en complexes de pectine-lignine-cellulose.

10. Procédé selon la revendication 1, **caractérisé en ce que** le petit- lait, avant addition de la substance pulvérulente, centrifugé à 4 °C pendant 45 minutes à 10 000 tr/min, est acidifié avec de l'acide citrique jusqu'à un pH de 5,50.

11. Procédé selon la revendication 1, **caractérisé en ce que** l'adsorbant granulaire est ajouté dans le petit-lait, dans un tampon de type phosphate à 0,12 mole.l⁻¹ à un pH de 4,50.

12. Procédé selon la revendication 1, **caractérisé en ce que** le précipité constitué d'adsorbant et de matières adsorbées, est traité successivement avec un tampon de type phosphate à 0,02 mole.l⁻¹ à un pH variant de 6,0 à 8,0 et du chlorure de sodium à 0,25 mole.l⁻¹, afin d'éliminer les substances étrangères souillant l'enzyme lactoperoxydase.

13. Procédé selon la revendication 1, **caractérisé en ce que** après élimination des substances étrangères selon la revendication 12, la lactoperoxydase est désorbée de l'adsorbant granulaire, par mise en suspension dans un tampon de type phosphate à 0,03 mole.l⁻¹ ayant un pH de 7,7, et une molarité en NaCl égale ou supérieure à 0,25.
